# EUROPEAN PATENT APPLICATION

(11) **EP 4 265 598 A1**
(43) Date of publication of application: **25.10.2023**
(21) Application number: 21903906.2
(22) Date of filing: 10.12.2021
(51) Int. Cl.: C07D 225/02, C08G 69/18

(54) **LAUROLACTAM PREPARATION METHOD, SYNTHESIZING DEVICE THEREFOR, LAUROLACTAM COMPOSITION PREPARED THEREBY, AND POLYLAUROLACTAM PREPARATION METHOD USING SAME**

(30) Priority: 11.12.2020 KR 20200173299
(71) Applicant: Hanwha Solutions Corporation, Jung-gu Seoul 04541 (KR)
(72) Inventor: SEO, Hyun, Daejeon 34128 (KR); KIM, Jiyeon, Daejeon 34128 (KR); JANG, Namjin, Daejeon 34128 (KR)
(74) Representative: Berggren Oy
(86) International application number: PCT/KR2021/018787
(87) International publication number: WO 2022/124861

(57) **Abstract**

The present invention relates to a laurolactam preparation method, a synthesizing device therefor, a laurolactam composition prepared thereby, and a polylaurolactam preparation method using the laurolactam composition, the laurolactam preparation method comprising the steps of: a) synthesizing cyclododecanone oxime into laurolactam through a Bechmann rearrangement in the presence of a catalyst; b) mixing the laurolactam synthesized in step a) in a good solvent and removing the catalyst; and c) mixing the laurolactam, from which the catalyst was removed in step b), in a poor solvent and recrystallizing same.

## Description

### [Technical Field]

The present invention relates to a laurolactam preparation method, a synthesizing device therefor, a laurolactam composition prepared thereby, and a polylaurolactam preparation method using the same.

In addition, the present invention provides a new laurolactam preparation method capable of solving a disadvantage that it is not easy to remove catalyst components from laurolactam prepared in a mixed catalyst of TCT (cyanuric chloride or trichlorotriazine) and ZnCl₂ that are used as a conventional catalyst and cocatalyst during preparation of the laurolactam.

### [Background Art]

In general, an industrial cyclic amide monomer, for example, laurolactam is synthesized through Bechmann rearrangement of cyclododecanone oxime. In general, industrially synthesized method of laurolactam, for example, as a cyclic amid monomer, cyclododecanone is synthesized through Bechmann rearrangement.

Polyamide-based heavies such as polylaurolactam may be synthesized by performing anionic polymerization, and since the purity of the laurolactam monomer has a great influence on the activity of an anionic polymerization reaction, the purity of the laurolactam monomer is a very important factor.

Conventionally, the laurolactam monomer prepared by the Bechmann rearrangement was purified by distilling and removing a solvent after completion of the Bechmann rearrangement and then removing heavies in a solid phase and/or a liquid phase, but there is still a problem that a small amount of catalyst remains in a final laurolactam product to rapidly kill activity of an anionic polymerization reaction.

Accordingly, methods of purifying laurolactam with high purity and increasing activity of anionic polymerization of a laurolactam monomer and the like by removing the catalyst remaining after the Bechmann rearrangement through a simplified process have been demanded.

As one of such methods, a method of recovering a monomer from which a catalyst is removed to some extent by synthesizing laurolactam from cyclododecanone oxime through Bechmann rearrangement under TCT and ZnCl₂ catalysts and adding a good solvent (ethanol, etc.) to the laurolactam to remove the catalyst from the laurolactam and then mixing a poor solvent (water) with the laurolactam again to recrystallize the laurolactam has been studied in Korean Patent Application No. 2019-161182 previously filed by the present applicant.

However, in Korean Patent Application No. 2019-161182, when the laurolactam is recrystallized by adding the poor solvent to the good solvent, sticky catalytic materials were still present at an interface. Therefore, even after the laurolactam is recrystallized, 90 ppm or more of catalyst was still present in the monomer to damage an anion reaction site during anionic polymerization of a recrystallized monomer, such that a degree of polymerization might not be increased or a conversion rate was decreased.

### [Disclosure]

### [Technical Problem]

An object of the present invention is to provide a new laurolactam preparation method capable of maintaining an amount of remaining catalyst at 20 ppm or less, preferably at 10 ppm or less, and more preferably at 1 ppm or less by preparing laurolactam from cyclododecanone oxime through Bechmann rearrangement and recrystallizing the synthesized laurolactam without sticky materials during good solvent and poor solvent treatment.

Another object of the present invention is to provide a synthesizing device for the laurolactam.

Still another object of the present invention is to provide a laurolactam in which a catalyst component synthesized by the laurolactam preparation method is maintained at 10 ppm or less.

Yet still another object of the present invention is to provide a polylaurolactam preparation method of preparing a polylaurolactam with a high conversion rate using an anionic polymerization reaction from the synthesized laurolactam.

### [Technical Solution]

In one general aspect, a laurolactam preparation method includes: a) synthesizing laurolactam from cyclododecanone oxime through Bechmann rearrangement under a TCT catalyst without using a cocatalyst; b) mixing the laurolactam synthesized in the step a) with a good solvent and removing the catalyst; and c) mixing the laurolactam from which the catalyst is removed in the step b) with a poor solvent to recrystallize the laurolactam.

In the step a), the Bechmann rearrangement may be to synthesize the laurolactam from the cyclododecanone oxime through a catalyst that only uses cyanuric chloride (TCT) and does not include zinc chloride (ZnCl₂), which is a cocatalyst used in Korean Patent Application No. 2019-161823 filed by the present applicant and not yet published, under a solvent including isopropylcyclohexane (IPCH).

The step a) may further include removing the solvent by distilling the synthesized laurolactam

In the step b), the catalyst may be removed using a difference in solubility of the good solvent between the catalyst and the laurolactam.

The good solvent may be a C1 to C4 hydrocarbon organic solvent containing one or two or more functional groups selected from the group consisting of a hydroxy group, an amine group, and a thiol group.

In the step c), the laurolactam may be recrystallized using a difference between solubility of the laurolactam in the good solvent and solubility of the laurolactam in the poor solvent.

The good solvent and the poor solvent may be miscible.

The poor solvent may be distilled water or deionized water.

The good solvent and the poor solvent may be injected at a weight ratio of 1:1.5 to 1:3.

The laurolactam preparation method may further include evaporating the recrystallized laurolactam to remove heavies in a liquid phase and/or a solid phase and separating the laurolactam in a gas phase.

In another general aspect, a synthesizing device for laurolactam includes: a first reactor synthesizing laurolactam from cyclododecanone oxime through Bechmann rearrangement under a cyanuric chloride (TCT) catalyst; an evaporator removing a solvent from the laurolactam synthesized in the first reactor; a second reactor mixing the laurolactam from which the solvent is removed in the evaporator with a good solvent and removing the catalyst; and a third reactor mixing the laurolactam from which the catalyst is removed in the second reactor with a poor solvent to recrystallize the laurolactam.

The synthesizing device for laurolactam may further include a filter removing the catalyst precipitated in the second reactor.

The synthesizing device for laurolactam may further include a film evaporator separating heavies from the recrystallized laurolactam.

In still another general aspect, a laurolactam composition synthesized by the laurolactam preparation method is provided.

The laurolactam composition may include the catalyst used in the Bechmann rearrangement in an amount of 5 wt% or less based on a total weight of the laurolactam composition.

In yet still another general aspect, a polylaurolactam preparation method of preparing a polylaurolactam by performing anionic polymerization of the laurolactam composition under an anionic initiator is provided.

The anionic initiator may include one or two or more selected from the group consisting of NaH, n-BuLi, KH, and LiH.

The anionic polymerization may be performed at 250 to 350°C for 10 to 60 minutes.

A weight average molecular weight of the polymerized polylaurolactam may exceed 6,000.

### [Advantageous Effects]

After laurolactam is synthesized through Bechmann rearrangement, a catalyst and a solvent remaining in a reaction product may be effectively removed only by a simplified process.

An anionic polymerization reaction may be performed with a high conversion rate using a purified laurolactam monomer.

### [Best Mode]

Hereinafter, the present invention will be described in detail. Terms used herein should be interpreted as the meanings generally understood by those skilled in the art unless otherwise defined. The drawings and examples of the present specification are for those skilled in the art to easily understand and practice the present invention, contents that may obscure the gist of the present invention in the drawings and examples may be omitted, and the present invention is not limited to the drawings and examples.

Singular forms of the terms used herein may be construed to include plural forms unless otherwise indicated.

In the specification of the present invention, a catalyst refers to a catalyst system that uses only a TCT catalyst and does not include a cocatalyst.

Hereinafter, a laurolactam preparation method according to an embodiment will be described.

The laurolactam preparation method includes a) synthesizing laurolactam from cyclododecanone oxime through Bechmann rearrangement under a TCT catalyst, b) mixing the laurolactam synthesized in the step a) with a good solvent and removing the catalyst, and c) mixing the laurolactam from which the catalyst is removed in the step b) with a poor solvent to recrystallize the laurolactam.

The step a) is a step of synthesizing the laurolactam from the cyclododecanone oxime through the Bechmann rearrangement under the TCT catalyst, wherein the Bechmann rearrangement may be to synthesize the laurolactam from the cyclododecanone oxime through a cyanuric chloride (TCT) catalyst in a solvent.

Specifically, in the step a), the Bechmann rearrangement may be performed at a temperature of 70 to 130°C, preferably 90 to 110°C, and more preferably 95 to 100°C, for 1 to 20 minutes, preferably 5 to 20 minutes, and more preferably 5 to 15 minutes. When a reaction temperature is excessively high in the Bechmann rearrangement, many byproducts such as heavies are generated, and when the reaction temperature is excessively low in the Bechmann rearrangement, a reaction speed is not sufficiently fast, such that it is difficult to apply the Bechmann rearrangement to a commercial process. In addition, when a reaction time is less than 1 minute, the cyclododecanone oxime may not be sufficiently rearranged into the laurolactam, and when the reaction time exceeds 20 minutes, an excessive side reaction occurs, which is not preferable.

Meanwhile, the Bechmann rearrangement refers to a rearrangement reaction in which ketoxime is converted into acid amide, and particularly in the present invention, the Bechmann rearrangement may refer to a reaction in which the cyclododecanone oxime is rearranged into the laurolactam.

The catalyst is cyanuric chloride (TCT). Specifically, the catalyst may be included in an amount of 0.1 to 10 parts by weight, preferably 0.1 to 5 parts by weight, and more preferably 0.5 to 2 parts by weight, based on 100 parts by weight of cyclododecanone oxime. When a content of the catalyst is excessively low, the Bechmann rearrangement may not be sufficiently performed, and when the content of the catalyst is excessively high, a high content of the catalyst material remains in a reaction product after the Bechmann rearrangement is completed, and thus, it is difficult to effectively remove the catalyst material in a purification step.

More specifically, the catalyst may include cyanuric chloride and zinc chloride in a weight ratio of 2:1 to 1:1, preferably 1.5:1 to 1:1, and more preferably 1.3:1 to 1:1. Accordingly, the laurolactam may be effectively synthesized by suppressing a conversion rate decrease phenomenon due to a moisture content limited in the Bechmann rearrangement, and the catalyst remaining in the reaction product after completion of the Bechmann rearrangement may be easily removed only by a simplified process.

The solvent is preferably an organic solvent including, for example, isopropylcyclohexane (IPCH). The solvent may be successfully used to prepare the laurolactam from the cyclododecanone oxime using the Bechmann rearrangement due to its strong non-polarity, and may be easily removed by distilling the reaction product due to a large difference in boiling point from the reaction product. Accordingly, the laurolactam may be effectively prepared with high purity.

The organic solvent including the IPCH may be used in an amount of 30 to 50 parts by weight based on 100 parts by weight of cyclododecanone oxime. When the organic solvent is included within the above content range, the Bechmann rearrangement of the cyclododecanone oxime is easy, and the solvent may be easily removed by distilling the reaction product. Accordingly, the laurolactam may be effectively prepared with high purity.

Subsequently, the step b) is a step of mixing the laurolactam synthesized in the step a) with the good solvent and removing the catalyst, wherein the good solvent may be a C1 to C4 hydrocarbon organic solvent, preferably a C1 to C4 hydrocarbon organic solvent containing one or two or more functional groups selected from the group consisting of a hydroxy group, an amine group, and a thiol group, and more preferably C1 to C4 hydrocarbon or C1 to C4 alcohol containing a hydroxyl group.

When the good solvent is mixed, the catalyst may be removed using a difference in solubility of the good solvent between the catalyst and the laurolactam. Specifically, the catalyst is not dissolved in the good solvent and is precipitated as solid particles, whereas the laurolactam has a high solubility in the good solvent, and may thus be substantially completely dissolved in the good solvent and be not precipitated. Thereafter, the catalyst precipitated as the particles may be easily removed with a filter. In this case, when an excessively small amount of good solvent is injected, some laurolactam may exist as a solid without being dissolved and may be removed together with the remaining catalyst with the filter, such that a yield of the laurolactam may be decreased, and some laurolactam that is not dissolved in the good solvent may aggregate with the remaining catalyst and pass through the filter to remain. Conversely, when an excessively large amount of good solvent is injected, it may not be easy to recrystallize the laurolactam in step c). Accordingly, the laurolactam synthesized in the step a) and the good solvent may be mixed with each other in a weight ratio of 1:4 to 1:7, preferably 1:5 to 1:7, and more preferably 1:6 to 1:7.

Meanwhile, in the specification of the present invention, the good solvent may refer to a solvent that may dissolve the laurolactam well due to its high affinity with the laurolactam, and the poor solvent may refer to a solvent that does not dissolve the laurolactam well due to its low affinity with the laurolactam.

Subsequently, the step c) is a step of mixing the laurolactam from which the catalyst is removed in the step b) with the poor solvent to recrystallize the laurolactam, wherein the poor solvent may be a material miscible with the good solvent, and may be specifically distilled water or deionized water. When the poor solvent is mixed, the laurolactam may be recrystallized using a difference between solubility of the laurolactam in the good solvent and solubility of the laurolactam in the poor solvent. Specifically, the laurolactam has high solubility in the good solvent and has low solubility in the poor solvent, and when the poor solvent is mixed with the good solvent in which the laurolactam is dissolved in the step b), as the concentration of the good solvent decreases,, the solubility of the laurolactam is decreased, and as a result, the laurolactam may be recrystallized and precipitated as a solid.

The good solvent and the poor solvent may be injected at a weight ratio of, for example, 1:1.5 to 1:3, preferably 1:2 to 1:3, and more preferably 1:2 to 1:2.5. When an excessive amount of the good solvent compared to the poor solvent is injected, some laurolactam may not be precipitated as the solid and may exist in a state in which it is dissolved in the good solvent, and a yield of the purified laurolactam may be decreased, which is not preferable.

Subsequently, a step of evaporating the recrystallized laurolactam to remove heavies in a liquid phase and/or a solid phase and separating the laurolactam in a gas phase may be further performed. As a result, the laurolactam may be purified with high purity, which is preferable.

The evaporation may be performed, for example, in a film evaporator, but the present invention is not limited thereto.

Meanwhile, the film evaporator is an evaporation device used to obtain a necessary material with high purity through a distillation reaction from a mixed material (liquid). That is, by forming a thin film from a liquid mixture by physical force to maximize a surface area of the liquid mixture, an evaporation rate may be increased and a material may be separated with high purity.

In addition, the present invention may provide a synthesizing device for laurolactam following the laurolactam preparation method described above. In this case, since the synthesizing device for laurolactam corresponds to substantially the same technical concept as the contents mentioned in the laurolactam preparation method, used materials, reaction conditions, and the like, should be interpreted to be substantially the same as the contents described above.

Hereinafter, a synthesizing device for laurolactam according to another embodiment will be described.

A synthesizing device for laurolactam according to the present invention includes a first reactor synthesizing the laurolactam from the cyclododecanone oxime through the Bechmann rearrangement reaction under the catalyst, an evaporator removing the solvent from the laurolactam synthesized in the first reactor, a second reactor mixing the laurolactam from which the solvent is removed in the evaporator with the good solvent and removing the catalyst, and a third reactor mixing the laurolactam from which the catalyst is removed in the second reactor with the poor solvent to recrystallize the laurolactam.

The synthesizing device for laurolactam may further include a filter removing a solid catalyst precipitated in the second reactor.

The synthesizing device for laurolactam may further include a film evaporator separating heavies from the recrystallized laurolactam.

Various known reactors, (film) evaporators, and filters may be used as the 'reactor', the '(film) evaporator', and the 'filter' mentioned in the present invention, and specifications and sizes of the 'reactors', the '(film) evaporator', and the 'filter' are not limited because they may be appropriately adjusted according to scales and environments of processes. In addition, various inlet pipes, outlet pipes, and the like, for introducing materials and discharging the materials may be provided in each reactor, (film) evaporator, and filter, and it may be appropriately adjusted by those skilled in the art to use various devices for adjusting an amount of introduced materials and an amount of discharged materials and various devices for controlling these devices.

Hereinafter, a laurolactam composition synthesized by the laurolactam preparation method according to another embodimentis provided.

A conversion rate of cyclododecanone oxime in the laurolactam composition may be 98 to 99%, preferably 99 to 99.5%, and more preferably 99.5 to 99.9%, and a selectivity of laurolactam in the laurolactam composition may be 97 to 98%, preferably 98 to 99%, and more preferably 99 to 99.5%.

In addition, the laurolactam composition may include the catalyst used in the Bechmann rearrangement in an amount of 5 wt% or less, preferably less than 5 wt%, more preferably 3 wt% or less, and most preferably 1 wt% or less or 0.5 wt% or less, based on a total weight of the laurolactam composition. If a content of the catalyst exceeds the above range, activity of an anionic initiator used in anionic polymerization is significantly killed, and thus, it is not easy to perform an anionic polymerization reaction.

Meanwhile, the content of the catalyst included in the laurolactam composition may be measured using an inductively coupled plasma spectrometer (ICP) analyzer.

Hereinafter, still another embodiment provides a polylaurolactam preparation method, and the polylaurolactam preparation method may include performing anionic polymerization of the laurolactam composition under an anionic initiator.

Polymerization of the purified laurolactam and any new monomer (comonomer) may be performed at 200 to 350°C for 10 to 60 minutes. Specifically, the polymerization reaction may be performed at 200 to 300°C and preferably 220 to 250°C for 10 to 60 minutes, preferably 10 to 50 minutes, and more preferably 20 to 40 minutes.

The polymerized polylaurolactam may be a laurolactam-containing polymer, for example, copolyamide or polyether-block amide, and preferably polyamide 12 (nylon 12).

The anionic initiator may specifically include one or two or more selected from the group consisting of NaH, LiH, KH, and n-BuLi. It has been known that the catalyst material used in the Bechmann rearrangement according to an embodiment of the present invention significantly kills the activity of the anionic initiator, but the laurolactam preparation method according to an embodiment of the present invention and the laurolactam composition prepared thereby very effectively remove the catalytic material, and accordingly, may perform anionic polymerization of the laurolactam monomer with a high degree of polymerization under the anionic initiator.

The anionic polymerization may be performed in a batch reactor or a continuous reactor (a continuous stirred tank reactor (CSTR), a plug flow reactor (PFR), or a packed-bed reactor (PBR)), and preferably in a continuous reactor, but the present invention is not limited thereto.

A weight average molecular weight of the polymerized polylaurolactam may exceed 6,000, and may be preferably 6,500 to 14,000, and more preferably 8,000 to 12,000 or 9,000 to 11,000.

Hereinafter, the present invention will be described in detail through examples, but these examples are provided in order to describe the present invention in more detail, and the scope of the present invention is not limited by the following examples.

### Example 1

3 g of cyclododecanone oxime, 12 g of isopropylcyclohexane, and 0.045 g of cyanuric chloride were injected into a 100 ml round flask. Then, a temperature was adjusted to 95°C using a heating mantle, and 3 g of cyclododecanone oxime, 12 g of isopropylcyclohexane, and 0.045 g of cyanuric chloride were stirred and reacted with each other at 200 rpm or more. A reaction completion time was 5 minutes, a conversion rate of the cyclododecanone oxime was 99% or more, and a selectivity of laurolactam was 99% or more.

100 g of product prepared above was injected into an evaporator and distilled at 150°C to remove isopropylcyclohexane (IPCH) through a top of the evaporator. 700 g of ethanol was injected into the resulting brown solid (laurolactam before being purified) and dissolved in the flask. A suspended solid (catalyst) was removed using a 0.22 um filter, and 1,600 g of water was injected into the laurolactam (LL) dissolved in the ethanol to recrystallize an LL solid. The recrystallized solid LL was separated using the filter, heavies were removed at a bottom using a film evaporator, the LL was separated at the top, and a content of remaining catalyst and a yield of the LL were measured and shown in Table 1 below. Sticky materials or materials that are not separated at an interface in the recrystallization were removed.

Subsequently, 50 g of prepared LL and a catalyst were injected into a 100 ml round flask at a weight ratio of LL:NaH:ethylene bis stearamide (EBS) :tetraethyl orthosilicate (TEOS):CO2 of 100:0.6:0.36:0.15:0.15, an anionic polymerization reaction was performed at 240°C for 30 minutes to prepare polyamide 12 (PA 12), and a degree of polymerization of PA 12 was shown in Table 1 below.

### Example 2

LL was separated in the same manner as in Example 1 except that 300 g of ethanol was injected, and a content of remaining catalyst and a yield of the LL were measured and shown in Table 1 below.

Subsequently, an anionic polymerization reaction was performed in the same manner as in Example 1 to prepare PA 12, and a degree of polymerization of PA 12 was shown in Table 1 below.

### Example 3

LL was separated in the same manner as in Example 1 except that 700 g of water was injected into the laurolactam (LL) dissolved in the ethanol, and a content of remaining catalyst and a yield of the LL were measured and shown in Table 1 below.

Subsequently, an anionic polymerization reaction was performed in the same manner as in Example 1 to prepare PA 12, and a degree of polymerization of PA 12 was shown in Table 1 below.

### Comparative Example 1

100 g of product of Preparation Example 1 was injected into an evaporator and distilled at 150°C to remove isopropylcyclohexane (IPCH) through a top of the evaporator. Heavies were removed from the resulting brown solid at a bottom using a film evaporator, the LL was separated at the top, and a content of remaining catalyst and a yield of the LL were measured and shown in Table 1 below.

Subsequently, an anionic polymerization reaction was performed in the same manner as in Example 1 to prepare PA 12, and a degree of polymerization of PA 12 was shown in Table 1 below.

### Comparative Example 2

LL was separated in the same manner as in Example 1 except that water was not injected into the laurolactam (LL) dissolved in the ethanol, and a content of remaining catalyst and a yield of the LL were measured and shown in Table 1 below.

Subsequently, an anionic polymerization reaction was performed in the same manner as in Example 1 to prepare PA 12, and a degree of polymerization of PA 12 was shown in Table 1 below.

### Comparative Example 3

The same processes as in Example 1 were performed except that 0.045 g of cyanuric chloride and 0.03 g of zinc chloride were injected as catalysts and reacted with each other. The results are shown in Table 1.

Subsequently, an anionic polymerization reaction was performed in the same manner as in Example 1 to prepare PA 12, and a degree of polymerization of PA 12 was shown in Table 1 below.

### * Method of measuring content of remaining catalyst

Since laurolactam is a solid at room temperature, the laurolactam is indistinguishable from a solid catalyst used during synthesis, but when the laurolactam is dissolved at 150°C, the catalyst remains as a black solid, and thus, it may be confirmed whether or not the solid catalyst remains. The catalyst remaining in the laurolactam prepared in Preparation Example 1 was separated as a solid at high temperature and a weight of the remaining catalyst was measured or a content of the remaining catalyst was measured using a solvent capable of dissolving the laurolactam.

### * Method of measuring yield of laurolactam

100 g of the product of Preparation Example 1 was measured by gas chromatography (GC) to calculate a content (L₁) of the laurolactam, and a content (L₂) of the laurolactam obtained at the top of the film evaporator of Example 1 was measured to calculate a yield (L₂/L₁ * 100) (%) of the laurolactam.

### * Method of measuring molecular weight (degree of polymerization) of PA12

In a polymerization reactor where an anionic polymerization reaction was completed, a stirrer torque value was calculated, and a weight average molecular weight of PA12 was calculated by inversely calculating the stirrer torque value.

### * Method of analyzing remaining catalyst

A content of Cl anions, which are remaining catalyst components, was analyzed through combustion ion chromatograph (IC) analysis. Ions generated by combustion of chlorine compounds by an Ar/O₂ gas and absorption of the chlorine compounds into a H₂O₂ solution were separated by an ion exchange column of an ion chromatograph, and quantitative analysis was then performed through a suppressor-detector.

**[Table 1]**

| | Laurolactam before being purified:eth anol (weight ratio) | Ethanol: water (weight ratio) | Content (wt%) of remaining catalyst | Degree of polymerization of PA 12 (weight average molecular weight) |
|---|---|---|---|---|
| Example 1 | 1:7 | 1:2.3 | 5ppm | 12,000 |
| Example 2 | 1:3 | 1:5.3 | 12ppm | 13,000 |
| Example 3 | 1:7 | 1:1 | 8ppm | 12,000 |
| Comp. Example 1 | - | - | 10,320ppm | not polymerized |
| Comp. Example 2 | 1:7 | - | 110ppm | 10,000 |
| Comp. Example 3 | 1:7 | | 94ppm | 10,000 |

Referring to Table 1, in Examples 1 to 3, the remaining catalyst was substantially removed, and PA12 could be prepared as a result of performing the anionic polymerization reaction using the purified laurolactam. However, in Comparative Example 3, in which a cocatalyst was used as a catalyst component in Example 1, 94 ppm of catalyst component still remained, and a molecular weight during anionic polymerization was also lower than a molecular weight of the present invention.

The present invention has been described hereinabove by specific matters and embodiments, but these specific matters and embodiments have been provided only in order to assist in a more general understanding of the present invention. Therefore, the present invention is not limited to these embodiments, and various modifications and alterations may be made from this description by those skilled in the art to which the present invention pertains. Accordingly, the spirit of the present invention should not be limited to these embodiments, and the claims and all of modifications equal or equivalent to the claims are intended to fall within the scope and spirit of the present invention.

## Claims

1. A laurolactam preparation method comprising:
a) synthesizing laurolactam from cyclododecanone oxime through Bechmann rearrangement under a cyanuric chloride (TCT) catalyst;
b) mixing the laurolactam synthesized in the step a) with a good solvent and removing the catalyst; and
c) mixing the laurolactam from which the catalyst is removed in the step b) with a poor solvent to recrystallize the laurolactam.

2. The laurolactam preparation method of claim 1, wherein in the step a), the Bechmann rearrangement is to synthesize the laurolactam from the cyclododecanone oxime through the cyanuric chloride (TCT) catalyst under a solvent including isopropylcyclohexane (IPCH).

3. The laurolactam preparation method of claim 2, wherein the step a) further includes removing the solvent by distilling the synthesized laurolactam.

4. The laurolactam preparation method of claim 1, wherein in the step b), the catalyst is removed using a difference in solubility of the good solvent between the catalyst and the laurolactam.

5. The laurolactam preparation method of claim 1, wherein the good solvent is a C1 to C4 hydrocarbon organic solvent containing one or two or more functional groups selected from the group consisting of a hydroxy group, an amine group, and a thiol group.

6. The laurolactam preparation method of claim 1, wherein in the step c), the laurolactam is recrystallized using a difference between solubility of the laurolactam in the good solvent and solubility of the laurolactam in the poor solvent.

7. The laurolactam preparation method of claim 1, wherein the good solvent and the poor solvent are miscible.

8. The laurolactam preparation method of claim 1, wherein the poor solvent is distilled water or deionized water.

9. The laurolactam preparation method of claim 1, wherein the good solvent and the poor solvent are injected at a weight ratio of 1:1.5 to 1:3.

10. The laurolactam preparation method of claim 1, further comprising evaporating the recrystallized laurolactam to remove heavies in a liquid phase and/or a solid phase and separating the laurolactam in a gas phase.

11. A synthesizing device for laurolactam, comprising:
a first reactor synthesizing laurolactam from cyclododecanone oxime through Bechmann rearrangement under a cyanuric chloride (TCT) catalyst;
an evaporator removing a solvent from the laurolactam synthesized in the first reactor;
a second reactor mixing the laurolactam from which the solvent is removed in the evaporator with a good solvent and removing the catalyst; and
a third reactor mixing the laurolactam from which the catalyst is removed in the second reactor with a poor solvent to recrystallize the laurolactam.

12. The synthesizing device for laurolactam of claim 11, further comprising a filter removing the catalyst precipitated in the second reactor.

13. The synthesizing device for laurolactam of claim 11, further comprising a film evaporator separating heavies from the recrystallized laurolactam.

14. A laurolactam composition synthesized by the laurolactam preparation method of any one of claims 1 to 10.

15. The laurolactam composition of claim 14, wherein the laurolactam composition includes the catalyst used in the Bechmann rearrangement in an amount of 5 wt% or less based on a total weight of the laurolactam composition.

16. A polylaurolactam preparation method of preparing a polylaurolactam by performing anionic polymerization of the laurolactam composition of claim 14 under an anionic initiator.

17. The polylaurolactam preparation method of claim 16, wherein the anionic initiator includes one or two or more selected from the group consisting of NaH, n-BuLi, KH, and LiH.

18. The polylaurolactam preparation method of claim 16, wherein the anionic polymerization is performed at 200 to 350°C for 10 to 60 minutes.

19. The polylaurolactam preparation method of claim 16, wherein a weight average molecular weight of the polymerized polylaurolactam exceeds 6,000.
